# EUROPEAN PATENT APPLICATION

(11) **EP 2 522 379 A1**
(43) Date of publication of application: **14.11.2012**
(21) Application number: 11165883.7
(22) Date of filing: 12.05.2011
(51) Int. Cl.: A61M 5/00, B65D 83/02, B65D 85/24, B65D 83/04

(54) **Needle assembly storage device**

(71) Applicant: Sanofi-Aventis Deutschland GmbH, 65929 Frankfurt (DE)
(72) Inventor: The designation of the inventor has not yet been filed

(57) **Abstract**

Described is a needle assembly storage device (1) comprising a case adapted to contain an array of needle assembly storage compartments (2) and a band (6) rotatably disposed on the case, the band (6) including an opening (8). Rotation of the band (6) aligns the opening (8) with an opening of a given one of the needle assembly storage compartments (2).

## Description

### Technical Field

The invention relates to a needle assembly storage device for needle assemblies that are usable with injection devices such as pen injectors and autoinjectors.

### Background of the Invention

Patients suffering from diseases like diabetes have to frequently self-administer injections. Injection devices like autoinjectors or pen injectors have been developed to facilitate self-administering injections. Typically, such injection devices are disposable or re-usable and refitted with sterile injection needle assemblies to minimize the risk of infections.

Portable needle assembly storage devices like needle magazines or needle dispensers contain a plurality of such sterile injection needle assemblies that are adapted to be mounted to the injection devices. The needle assembly storage devices supplement the injection devices to facilitate safe self-administration of the medicament. Additionally, the needle assembly storage device may be used as a disposal container for used injection needle assemblies to reduce the risk of accidental needle stick injuries caused by contaminated injection needles.

US 7,134,550, US 5,873,462 and WO 2009/136193 discuss portable needle assembly storage devices. However, there remains a need for a needle assembly storage device to allow for easy transportation and access to a plurality of needle assemblies and allow for disposal of used needle assemblies.

### Summary of the Invention

It is an object of the present invention to provide an improved means for storing and disposing needle assemblies

In an exemplary embodiment, a needle assembly storage device comprises a case adapted to contain an array of needle assembly storage compartments and a band rotatably disposed on the case. The band includes an opening, and rotation of the band aligns the opening with an opening of a given one of the needle assembly storage compartments.

In an exemplary embodiment, the case comprises a pair of side walls, and the band is disposed between the side walls. Each of the side walls may include a bearing surface formed on an inner surface, and the band may be disposed on the bearing surface of each of the side walls.

In an exemplary embodiment, a handle is disposed on the band.

In an exemplary embodiment, a slot is formed in the case and adapted to receive a barrier strip from the given one of the needle assembly storage compartments. A container may be coupled to the slot and adapted to store the barrier strip.

In an exemplary embodiment, the band includes an inner surface having a plurality of retention ramps and each of the retention ramps including an abutment face. A hook may be disposed in the case, and a given one of the retention ramps can deflect the hook when the band moves in a first rotational direction and the abutment face abuts the hook preventing movement of the band in a second rotational direction. An audible feedback may be generated when the hook disengages the given one of the retention ramps.

In an exemplary embodiment, the band is adapted to incrementally rotate a predetermined step distance. The predetermined step distance may be substantially equal to a width of the given one of the needle assembly storage compartments. In another exemplary embodiment, the predetermined step distance is substantially equal to half of a width of the given one of the needle assembly storage compartments.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
Figure 1 shows a lateral view of a needle assembly storage device according to an exemplary embodiment of the present invention,
Figure 2 shows front view of a needle assembly storage device according to an exemplary embodiment of the present invention,
Figure 3 shows a sectional view of a needle assembly storage device according to an exemplary embodiment of the present invention,
Figure 4 shows an array of needle assembly storage compartments according to an exemplary embodiment of the present invention, and
Figure 5 shows a front view of a needle assembly storage device with the array of needle assembly storage compartments shown in figure 4.

Corresponding parts are marked with the same reference symbols in all figures.

### Detailed Description

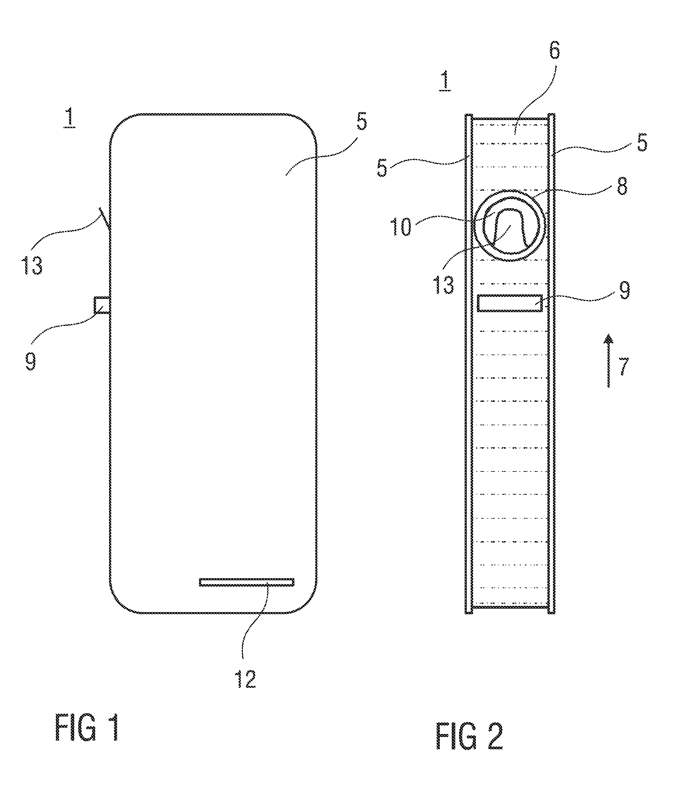

Figures 1, 2 and 3 show a lateral view, a front view and a sectional view of an exemplary embodiment of a needle assembly storage device 1, respectively, according to the present invention. The needle assembly storage device 1 comprises a case and a plurality of needle assembly storage compartments 2 arranged inside the case. The needle assembly storage compartments 2 may be linked together to form an array, which may be manufactured by a single injection moulding process. Each of the needle assembly storage compartments 2 is suited to contain a needle assembly 3 with a needle 4 mounted on a needle support 14. The needle assembly 3 may be adapted to be removably coupled to an injection device, e.g., pen injector, autoinjector, etc.

In an exemplary embodiment, the case comprises two opposing side walls 5 and a band 6 which is rotatable relative to the side walls 5 in a first rotational direction 7. The band 6 surrounds a space between the side walls 5 and has an opening 8 shaped and sized to give access to one of the needle assembly storage compartments 2. That is, when the opening 8 is aligned within an opening of a needle assembly storage compartment 2, an injection device can be inserted into the needle assembly storage compartment 2 to engage an unused needle assembly and to deposit a used needle assembly. In an exemplary embodiment, the case may be circular or ellipsoidal, but those of skill in the art will understand that the case may be any shape or size.

In an exemplary embodiment, a handle 9 may be disposed on the band 6 or case to allow a user to rotate the band 6 to gain access to the needle assembly storage compartments 2. In another exemplary embodiment, a lever, wheel or crank may be utilized to rotate the band 6.

In an exemplary embodiment, at least one of the side walls 5 may be removably or hingedly coupled to the case. After all of the needle assemblies 3 in the array have been used, the case may be opened and the used needle assemblies (or the array of used needle assemblies) may be discarded. An array of unused needle assemblies may then be inserted into the case. The case may further include a lock for locking at least one of the side walls 5 to the case.

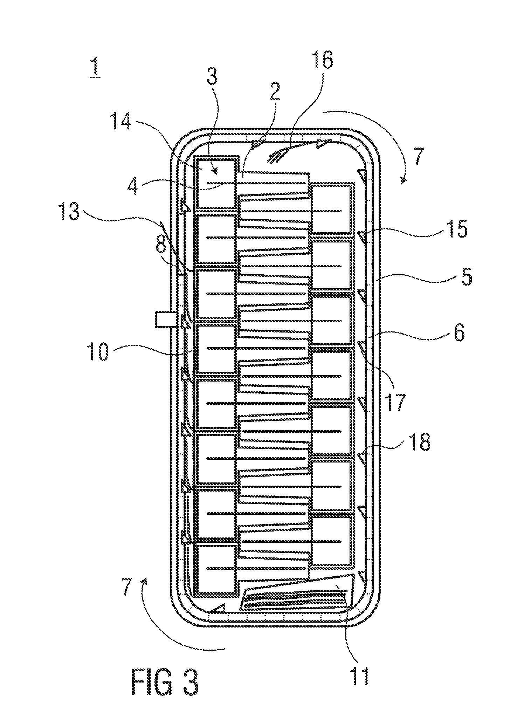

As shown in Figure 1, the case may include a slot 12 for receiving a barrier strip 10 that was removed from a needle assembly storage compartment 2. The slot 12 may provide an opening into a container 11 formed in the case (shown in Figure 3). The barrier strip 10 may be disposed over an opening of the needle assembly storage compartment 2 to maintain sterility of the needle assembly 3 therein. Prior to use of a needle assembly 3 in a needle assembly storage compartment 2, the barrier strip 10 may be removed (e.g., by pulling on tab 13) and inserted into the slot 12. The slot 12 may be useful when, for example, the user is in a location in which he/she does not know a location of a trash receptacle.

As the band 6 is rotated in the first rotational direction 7, the user will see the opening 8 move over the openings of the needle assembly storage compartments 2. As the opening 8 becomes aligned with the opening of a needle assembly storage compartment 2, the tab 13 of the barrier strip 10 may be exposed, allowing the user to manually remove the barrier strip 10. In another exemplary embodiment, a removal hook may be disposed on the band 6 adjacent the opening 8. When the band 6 is rotated in the first rotational direction 7, the removal hook may engage and hold the tab 13 and separate the tab 13 from the needle assembly storage compartment 2 as the band 6 continues to rotate.

As shown in the exemplary embodiment of Figure 3, the array of needle assembly storage compartments 2 may be configured such that the openings of consecutive needle assembly storage compartments face in opposite directions. For example, each needle assembly storage compartment 2 may have a cylindrical portion for receiving the needle support 14 of the needle assembly 3 and a conical portion, extending from the cylindrical portion, for encasing the needle 4. In the exemplary embodiment depicted in Figure 3, the conical portions of consecutive needle assembly storage compartments 3 abut each other, and the cylindrical portions of every other needle assembly storage compartment abut each other. In this exemplary orientation, the casing can be shorter and more portable than if the needle assembly storage compartments 3 were oriented to face in the same direction.

In an exemplary embodiment, the band 6 may be rotated relative to the side walls 5 to align the opening 8 with an opening of a given one of the needle assembly storage compartments 2. Each of the side walls 5 may include an inner surface having a track and one or more connectors formed thereon. The track may be formed as a ridge or projection which provides a bearing surface for a lateral edge of the band 6. When the band 6 is placed on the case, the tracks on the side walls 5 may provide bearing surfaces to support the band 6 and allow it to rotate relative to the side walls 5. The connectors may be used to connect the side walls 5. For example, one side wall 5 may include male connectors and the other side wall 5 may include female connectors. In an exemplary embodiment, the connectors are disposed internal to the tracks on the side walls 5.

In an exemplary embodiment, the band 6 may be rotatable only in the first rotational direction 7. An inner surface of the band 6 may include a plurality of retention ramps 15, each of which are adapted to abut a hook 16 if there is an attempt to rotate the band 6 in a second rotational direction opposite the first rotational direction. A distance between consecutive retention ramps 15 may be substantially equal to a width of a needle assembly storage compartment 2 (and in particular, substantially equal to a width of an opening of a needle assembly storage compartment 2). The hook 16 may be resiliently disposed in the case, e.g., by projecting from an inner surface of one of the side walls 5 into an interior of the case. As the band 6 is rotated in the first rotational direction 7, an angular face 17 of a retention ramp 15 engages the hook 16, causing the hook 16 to deflect and allowing the retention ramp 15 to pass (after which the hook 16 returns to its original, non-deflected position). An audible feedback (e.g., a "snap" or "click" sound) may be provided when the retention ramp 15 passes the hook 16, for example when the hook 16 disengages the angular face 17 of the retention ramp 15. If the band 6 is rotated in the second rotational direction, an abutment face 18 on the retention ramp 15 abuts the hook 16 and prevents the band 6 from being rotated further in the second rotational direction. Preventing rotation in the second rotational direction may prevent the user from reusing used needle assemblies which have been returned to the needle assembly storage compartments.

The band 6 may be configured to rotate a predetermined step distance. In an exemplary embodiment, the step distance may be substantially equal to a width of a needle assembly storage compartment 2 (and in particular, substantially equal to a width of an opening of a needle assembly storage compartment 2). In this exemplary embodiment, the step distance corresponds to the distance between consecutive retention ramps 15 formed on the band 6. In another exemplary embodiment, the step distance may be substantially equal to half of the width of a needle assembly storage compartment 2 (and in particular, substantially equal to half of the width of an opening of a needle assembly storage compartment 2). In this exemplary embodiment, the user may rotate the band 6 by the step distance after depositing a used needle assembly into its needle assembly storage compartment, which may partially obstruct consecutive needle assembly storage compartments. This may prevent exposure of a needle in the used needle assembly and may be useful when transporting the needle assembly storage device 1.

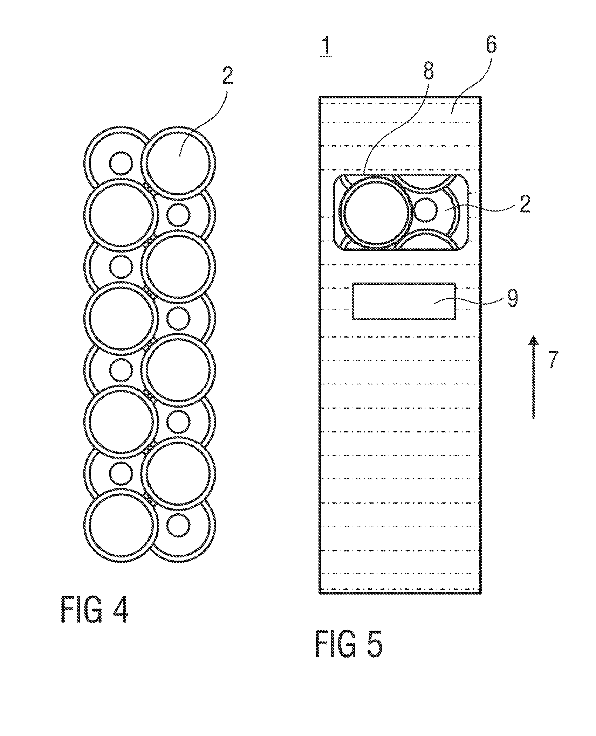

Figure 4 shows another exemplary embodiment of an array of needle assembly storage compartments 2. In this exemplary embodiment, the array may be two-dimensional having consecutive needle assembly storage compartments 2 disposed in two planes and at an angle to each other. For example, the consecutive needle assembly storage compartments 2 may be disposed at a 45 degree angle relative to each other. Additionally, the array may be formed of two rows of the needle assembly storage compartments 2, and the storage compartments 2 in a first row may be inverted relative to the storage compartments in a second row (as in the exemplary embodiment described above with reference to Figure 3).

Figure 5 shows an exemplary embodiment of a needle assembly storage device 1 according to the present invention for accommodating the exemplary embodiment of the array depicted in Figure 4. In this exemplary embodiment, the opening 8 may be wider than a width of an opening of a needle assembly storage compartment 2, e.g., to accommodate the two planes of needle assembly storage compartments 2. Additionally, the band 6 may be rotatable in a step distance which is substantially equal to half of the width of a needle assembly storage compartment, thereby providing access to all of the needle assembly storage compartments 2.

Those of skill in the art will understand the modifications (additions and/or removals) of various components of the device and/or system and embodiment described herein may be made without departing from the full scope and spirit of the present invention, which encompass such modifications and any and all equivalents thereof.

### List of References

- 1: needle assembly storage device
- 2: needle assembly storage compartment
- 3: needle assembly
- 4: needle
- 5: side wall
- 6: band
- 7: first rotational direction
- 8: opening
- 9: handle
- 10: barrier strip
- 11: container
- 12: slot
- 13: tab
- 14: needle support
- 15: retention ramp
- 16: hook
- 17: angular face
- 18: abutment face

## Claims

1. A needle assembly storage device (1) comprising:
a case adapted to contain an array of needle assembly storage compartments (2); and
a band (6) rotatably disposed on the case, the band (6) including an opening (8),
wherein rotation of the band (6) aligns the opening (8) with an opening of a given one of the needle assembly storage compartments (2).

2. The needle assembly storage device (1) according to claim 1, wherein the case comprises:
a pair of side walls (5),
wherein the band (6) is disposed between the side walls (5).

3. The needle assembly storage device (1) according to claim 2, wherein each of the side walls (5) includes a bearing surface formed on an inner surface, and the band (6) is disposed on the bearing surface of each of the side walls (5).

4. The needle assembly storage device (1) according to any of the preceding claims, further comprising:
a handle (9) disposed on the band (6).

5. The needle assembly storage device (1) according to any of the preceding claims, further comprising:
a slot (12) formed in the case and adapted to receive a barrier strip (10) from the given one of the needle assembly storage compartments (2);
a container (11) coupled to the slot (12) and adapted to store the barrier strip (10).

6. The needle assembly storage device (1) according to any of the preceding claims, wherein the band (6) includes an inner surface having a plurality of retention ramps (15), each of the retention ramps (15) including an abutment face (18).

7. The needle assembly storage device (1) according to claim 6, further comprising:
a hook (16) disposed in the case,
wherein a given one of the retention ramps (15) can deflect the hook (16) when the band (6) moves in a first rotational direction and the abutment face (18) abuts the hook (16) preventing movement of the band (6) in a second rotational direction.

8. The needle assembly storage device (1) according to claim 7, wherein an audible feedback is generated when the hook (16) disengages the given one of the retention ramps (15).

9. The needle assembly storage device (1) according to any of the preceding claims, wherein the band (6) is adapted to incrementally rotate a predetermined step distance.

10. The needle assembly storage device (1) according to claim 9, wherein the predetermined step distance is substantially equal to a width of the given one of the needle assembly storage compartments (2).

11. The needle assembly storage device (1) according to claim 9, wherein the predetermined step distance is substantially equal to half of a width of the given one of the needle assembly storage compartments (2).
